# EUROPÄISCHE PATENTSCHRIFT

(11) **EP 0 591 136 B1**
(45) Veröffentlichungstag und Bekanntmachung des Hinweises auf die Patenterteilung: **29.11.1995**
(21) Anmeldenummer: 90912042.0
(22) Anmeldetag: 14.08.1990
(51) Int. Cl.: A61M 15/00

(54) **INHALATOR**
INHALATOR
INHALATEUR

(30) Priorität: 17.08.1989 DE 3927170
(43) Veröffentlichungstag der Anmeldung: 13.04.1994
(73) Patentinhaber: BOEHRINGER INGELHEIM INTERNATIONAL GmbH, 55218 Ingelheim am Rhein (DE); BOEHRINGER INGELHEIM KG, 55216 Ingelheim (DE)
(72) Erfinder: GUPTE, Arun, Rajaram, D-6507 Ingelheim (DE); HERTL, Erich, D-8031 Gilching (DE)
(86) Internationale Anmeldenummer: EP9001336
(87) Internationale Veröffentlichungsnummer: WO9102558

(56) Entgegenhaltungen:
- EP-A- 0 147 755
- DE-A- 2 531 618
- US-A- 3 870 046
- US-A- 3 918 451
- US-A- 4 046 146
- Derwent's abstract, No. 84-268 035/43, SU 1 076 132, publ. week 8443 (SIDORENKO YO S)

## Beschreibung

Die Erfindung betrifft einen Inhalator für die Inhalation pulverförmiger, insbesondere mikronisierter Arzneimittel aus Kapseln, in dessen Gehäuse für die Aufnahme und Halterung der Kapseln eine rohrförmige Kammer mit einem bodenseitigen Lufteinlaß und einem in ein Inhalationsmundstück übergehenden Luftauslaß am gegenüberliegenden Kammerende und eine Schneideinrichtung mit zwei in den Kammerinnenraum bewegbaren Schneiden zum Öffnen der Kapseln in der Nähe von deren oberen und unteren Ende angeordnet sind.

Die US-PS 2 569 720 beschreibt einen Inhalator mit einer Kammer, in der pulverförmige Arzneimittel verteilt sind, und die beim Inhalieren von Luft durchströmt wird. Um zu verhindern, daß das Arzneimittelpulver in das mit dem Luftauslaß der Kammer verbundene Mundstück gerät, ist dort ein Sieb mit geeigneter Maschenweite vorgesehen. Nachteilig bei diesem Inhalator ist jedoch, daß die das Arzneimittel enthaltene Kapsel, die meist aus Hartgelatine besteht, vor Inbetriebnahme per Hand geöffnet und das pulverförmige Arzneimittel - mit oder ohne Kapsel - in die Kammer eingeführt werden muß; anschließend muß der Inhalator jeweils erst zusammengesetzt werden.

Die US-PS 3 918 451 betrifft einen Inhalator, dessen Gehäusemantel eine Öffnung besitzt, die durch Drehung des Gehäusemantels um die Gehäuselängsachse in eine derartige Lage gebracht werden kann, daß die Kammer seitlich frei zugänglich ist und ein Bestücken bzw. Reinigen des Kammerinnenraums möglich wird. Nach Einlegen der Kapsel wird die Kammer durch Drehung des Gehäusemantels verschlossen, bevor zwei Schneiden einer Schneideinrichtung betätigt werden, die zur Öffnung der Kapsel am oberen und unteren Ende dienen.

Weiterhin beschreibt die US-PS 4 069 819 einen Inhalator, dessen Kammer so ausgestaltet sein soll, daß eine darin befindliche Kapsel von der durchströmenden Luft in Rotation, Auf- und Abbewegung und Vibration versetzt wird, um eine gleichmäßige Verteilung des Arzneimittelpulvers zu gewährleisten.

Auf demselben Prinzip beruht auch der in der EP 0 147 755 A2 beschriebene Inhalator. Seine Kammer ist im wesentlichen zylindrisch und besitzt jeweils koaxial am unteren bzw. oberen Ende einen Lufteinlaß und einen Luftauslaß, deren Öffnungen jeweils kleiner als der Kapseldurchmesser sind. Dieser Inhalator ermöglicht nicht nur eine verläßlichere Ausbringung des Arzneimittels mit geringer Standardabweichung, sondern er hat auch den zusätzlichen Vorteil, daß er bei Betätigung das Pulver der Kapsel besser desagglomeriert. Nachteilig bei diesem wie auch bei den vorher beschriebenen Inhalatoren ist jedoch, daß vor bzw. nach jedem Inhalationsvorgang die einzige Kammer entleert bzw. gereinigt werden muß, was ein vorheriges Öffnen bzw. Auseinandernehmen der lösbaren oder verschiebbaren Gehäuseteile erfordert.

Es ist daher Aufgabe der vorliegenden Erfindung, den eingangs beschriebenen Inhalator dahingehend weiterzuentwickeln, daß seine Handhabung wesentlich vereinfacht wird, insbesondere ohne großen technischen Aufwand die Einbringung der Kapsel vor dem jeweils nächsten Inhalationsvorgang in die rohrförmige Kammer erleichtert wird und ein sofort verwendbarer Kapselvorrat zur Verfügung steht.

Diese Aufgabe wird durch den im Patentanspruch 1 beschriebenen Inhalator gelöst. Erfindungsgemäß ist statt einer einzelnen Kammer, in die jweils eine Kapsel eingelegt bzw. aus der die Kapsel herausgenommen werden muß, ein Revolvermagazin vorgesehen, das mehrere mit jeweils einer Kapsel bestückte rohrförmige Kammern besitzt, die jeweils zwischen den Luftein- und auslaß eingeschwenkt werden können und somit einen Teil des Inhalationsluft-Durchgangskanales bilden. Nach Beendigung eines Inhalationsvorganges wird das Revolvermagazin weitergedreht, bis die nächstfolgende Kammer in den Luftdurchgangskanal kommt. Ein Öffnen des Gehäuses bzw. ein aufwendiger Kapselaustausch oder eine Reinigung der Kammer entfällt. Weiterhin besitzt das Gerät den Vorteil, daß es mit einer Hand bedient werden kann.

Nach einer Weiterbildung der Erfindung ist das Revolvermagazin lösbar im Inhalatorgehäuse angeordnet. Nach Verbrauch der im Revolvermagazin vorhandenen Kapseln kann somit das komplette Revolvermagazin ausgetauscht oder neu mit Kapseln gefüllt werden.

Nach einer Weiterbildung der Erfindung besitzt das Inhalatorgehäuse einen exzentrisch angeordneten Stift, auf den das Revolvermagazin aufsteckbar ist.

Zur Fixierung der Position des Revolvermagazins kann man es mit den Kapselkammern jeweils zugeordneten Ausnehmungen für einen in dem Inhalatorgehäuse angeordneten federnd gelagerten Arretierbolzen versehen. Die Ausnehmungen sind so angeordnet, daß der Arretierbolzen nur dann dort einrastet, wenn eine der Kapselkammern sich genau zwischen Luftein- und -auslaß befindet.

Damit kann sichergestellt werden, daß sich das Revolvermagazin während der Inhalation nicht verschiebt. Die federnde Lagerung des Arretierbolzens sollte hinsichtlich der Federkonstanten so gewählt werden, daß ein versehentliches Verdrehen des Revolvermagazins durch die Arretierung verhindert, andererseits bei stärkerer Krafteinwirkung das Revolvermagazin aus der Arretierung herausgedreht werden kann. Konische Ausgestaltungen des freien Endes des Arretierbolzens und entsprechend geformte Ausnehmungen wirken hierbei unterstützend.

Der Arretierbolzen ist vorzugsweise koaxial zum Luftdurchgangskanal unter der Kapselkammer angeordnet und weist eine Durchgangsbohrung auf, die gleichzeitig den bodenseitigen Lufteinlaß bildet. Vorzugsweise ist der Arretierbolzen zentrisch im Inhalatorgehäuse angeordnet. Nach einer weiteren Ausgestaltung der Erfindung wird der Arretierbolzen durch eine Feder beaufschlagt, deren anderes Ende auf einem im Inhalatorgehäuse lösbar befestigten Stopfen aufliegt, der ebenfalls eine zentrale Durchgangsbohrung aufweist, die Teil des Luftdurchgangskanals ist.

Die Höhe der Kapselkammer ist durch die Länge der Arzneimittelkapseln bestimmt. Dementsprechend sind auch die Schneiden der Schneideinrichtung, die gegen den Druck einer Feder ins Kammerinnere verschiebbar sind, im Bereich des oberen und unteren Endes der Kapselkammer angeordnet. Die Seitenwand von jeder Kapselkammer weist im Bereich ihres oberen und unteren Endes den Schneiden zugewandte radiale äußere Durchbrechungen oder zumindest geschwächte Bereiche auf, die als Durchführungen für die Schneiden dienen.

Das Mundstück des Inhalators ist, wie bereits gesagt, als Kappe ausgebildet, die auf das Unterteil des Inhalators aufgesetzt ist. Sie kann am Inhalatorgehäuserand um eine senkrecht zur Inhalatorlängsachse liegende Achse schwenkbar angelenkt sein. Mundstück und Unterteil des Inhalatorgehäuses können aber auch durch eine übliche Steckverbindung aneinander befestigt sein. Durch die Lösbarkeit bzw. Verschwenkbarkeit der beiden Teile ist jedenfalls der Zugang insgesamt, einerseits zum Revolvermagazin und der Schneidvorrichtung im Gehäuseunterteil und andererseits zu den innen liegenden Teilen, wie der Siebplatte, des Gehäuseoberteiles (der mundstückartigen Kappe) wesentlich vereinfacht.

Zum Austausch der verbrauchten Kapseln gegen frische wird das Mundstück hochgeklappt oder die Steckverbindung zwischen Mundstück und Gehäuseunterteil gelöst. Die Kammern des Magazins sind dann frei zugänglich, so daß die entleerten Kapseln entnommen und gefüllte eingelegt werden können. Das Magazin kann jedoch auch abgezogen und nach Entleerung mit frischen Kapseln gefüllt wieder aufgesteckt werden. Sodann wird das Gerät zugeklappt bzw. zusammengesteckt. Im oberen Bereich der Kammer, dort, wo sie in den Inhalationskanal übergeht, ist eine Siebplatte angeordnet, die Teil eines trichterförmigen Verbindungsstückes ist, welches auf den Anfang des Inhalationskanales so aufsteckbar ist, daß der Trichterrand mit der Siebplatte in eine Einsatzplatte eingreift, die den Boden des Mundstückes bildet. Die Siebplatte kann aber auch im Klemmsitz zwischen dem Trichterrand des Verbindungsstückes und einem Anschlag der Einsatzplatte austauschbar befestigt sein. Sie verhindert, daß eine Kapsel beim Inhalieren die Luftaustrittsöffnung verschließt oder daß eventuell entstandene Kapselbruchstücke in das Mundstück gesaugt werden.

In einer bevorzugten Ausführungsform sind die Ausnehmungen für den Eingriff des Arretierbolzens bodenseitig in der Bodenplatte des Magazins konzentrisch zu den Lufteintrittsbohrungen der Kapselkammern angeordnet und wie der Mantel eines mit der Basis nach außen gewandten flachen Kegelstumpfes gestaltet. Es handelt sich also bei diesen Ausnehmungen um konische bzw. trichterförmige Erweiterungen der Lufteintrittsbohrungen, wobei der erweiterte Bereich dem Arretierbolzen zugewandt ist. Die durch die Erweiterung entstehenden Schrägen entsprechen in etwa den Abschrägungen am Kopf des Arretierbolzens.

In einer bevorzugten Ausführungsform weisen diese Ausnehmungen an der Basis des Kegelstumpfmantels, aber noch in der Bodenplatte, eine umlaufende Anschlagskante auf, die als Verdrehsicherung bzw. Anschlag für den Kopf des Arretierbolzens dient, wenn dieser in die Ausnehmung eingerastet ist. Aufgrund der besagten Anschlagskante kann man also bei eingerastetem Arretierbolzen das Magazin nicht weiterdrehen.

In einer anderen Ausgestaltung dieser Ausführungsform nimmt die besagte Anschlagskante nur einen Teil oder die Hälfte des Umfangs der konischen Ausnehmung, also der trichterförmigen Erweiterung, ein und ist so angeordnet, daß sie das Verdrehen des Magazins bei eingerastetem Arretierbolzen in einer Richtung sperrt, in der anderen Richtung aber zuläßt, weil dort die schräge Wand der trichterförmigen Erweiterung der Ausnehmung glatt in die Außenseite der Bodenplatte übergeht.

In einer weiteren bevorzugten Ausführungsform weist nur eine der Ausnehmungen eine den ganzen Umfang der Ausnehmung einnehmende Anschlagskante auf, so daß in dieser Ausnehmung bei eingerastetem Arretierstift ein Verdrehen des Magazins nicht möglich ist. Diese Position betrachtet man dann als Endposition eines Magazins, in dem alle Kapseln verbraucht sind. Alle anderen Ausnehmungen weisen bei dieser Ausführungsform nur die einseitige, d.h. in einer Richtung wirkende Verdrehungssperre auf, so daß das Magazin immer nur in Richtung des Einschwenkens einer Kapselkammer mit einer unverbrauchten Kapsel gedreht werden kann, bis die zuvor geschilderte Endposition erreicht ist, in der die Arretierung vollständig ist. Der Benutzer weiß dann, daß das Magazin mit frischen Kapseln zu beschicken ist, wenn diese letzte Kapsel verbraucht ist.

In einer anderen bevorzugten Ausführungsform sind die Schneiden der Schneideinrichtung in einer federnd gelagerten Dichtungsplatte geführt. Auf diese Weise wird die Dichtung zwischen der in Inhalationsposition befindlichen Kapselkammer und der Schneidvorrichtung verbessert. Für die federnde Lagerung der Dichtungsplatte kann die Feder verwendet werden, welche das Rückstellen der Betätigungstaste der Schneidvorrichtung bewirkt.

In einer weiteren bevorzugten Ausführungsform kann am Arretierbolzen eine Zunge befestigt sein, die sich bis zu einem Anschlag an der Innenseite der Bedienungstaste der Schneidvorrichtung erstreckt, wenn der Arretierbolzen bei entnommenem Revolvermagazin seine obere Anschlagsposition einnimmt. Die besagte Zunge wirkt in dieser Position als Sperre für die Schneideinrichtung. Beim Einsetzen des Magazins wird der Arretierbolzen wieder nach unten gedrückt und damit auch die Sperre der Schneidvorrichtung beseitigt.

Schließlich ist in einer weiteren bevorzugten Ausführungsform des Inhalators ein Hebelsystem für die Betätigung der Schneideinrichtung vorgesehen. Dieses Hebelsystem wird vorzugsweise von einer am Boden des Gehäuses angebrachten Betätigungstaste aus betätigt. Das Hebelsystem kann aus einer Wippe und einem Kniehebel bestehen, wobei auf das eine Ende der Wippe die Betätigungstaste einwirkt, und das andere Ende der Wippe auf das eine Ende des Kniehebels drückt, wobei das an der Schneidvorrichtung befestigte andere Ende des Kniehebels die Schneidvorrichtung vorschiebt. Wippe und Kniehebel sind vorzugsweise um Achsen schwenkbar in Halterungen gelagert, die am Gehäuse befestigt sind. Die Betätigung der Schneidvorrichtung über das besagte Hebelsystem kann auch mit der Drehbewegung des Kapselmagazins gekoppelt werden, so daß mit einem Tastendruck zuerst eine Kapselkammer in die richtige Position gebracht wird und anschließend sofort die Schneidvorrichtung angreift.

Werden das Revolvermagazin und der an dieses angrenzende Teil des Inhalatorgehäuses n--eckig ausgestaltet, wobei n eine ganze, die Anzahl der Kapselkammern angebende Zahl ist, so läßt man vorteilhaft die Seitenflächen des Inhalatorgehäuseteiles und des Revolvermagazins fluchten, wenn das Magazin in der richtigen Position ist. Man kann dann unmittelbar von außen feststellen, ob die Kammer in dem durch den Lufteinlaß und den Luftauslaß definierten Luftkanal liegt.

Nach einer Weiterbildung der Erfindung hat der Inhalator unter dem Revolvermagazin mindestens einen Hohlraum mit Durchbrechungen nach oben und nach unten.

Die Durchbrechungen nach unten stellen eine Verbindung zum bodenseitigen Lufteinlaß her. Durch diese Ausführungsform ist gewährleistet, daß beim Inhalieren hinreichend viel Luft zum Lufteinlaß gelangt.

Vorzugsweise ist die lichte Weite der Kapselkammern etwa 1,1 bis 2mal so groß wie der Kapseldurchmesser und die gesamte Länge der Kapselkammer etwa 1,1 bis 1,6 mal so groß wie die Kapsellänge, wobei die lichte Weite kleiner sein muß als die Länge der Kapsel, um ein Umkippen der Kapsel zu verhindern. Selbstverständlich ist eine Halterung der Kapsel im Revolvermagazin nur möglich, wenn die Lufteintrittsöffnung im Boden des Hohlraumes kleiner als der Kapseldurchmesser ist. Nach oben hin können die Hohlräume offen sein, einen ebenfalls sich verjüngenden Luftaustritt aufweisen oder mit einer Siebplatte abgedeckt sein.

Ausführungsformen der Erfindung sind in den Zeichnungen dargestellt. Es zeigen
- Fig. 1a, 1b: jeweils Seitenansichten des Inhalatorgehäuses mit aufgeklapptem Mundstück und des Revolvermagazins,
- Fig. 2a, 2b: jeweils Draufsichten nach Fig. 1,
- Fig. 2c: eine Ansicht der Bodenplatte des Revolvermagazins,
- Fig. 2d, 2e: jeweils vergrößerte Ausschnitte der Einraststelle des Arretierbolzens,
- Fig. 3: einen Schnitt durch eine erste Ausführungsform des Inhalators,
- Fig. 4: einen Schnitt durch eine zweite Ausführungsform des Inhalators,
- Fig. 5: einen Schnitt durch eine dritte Ausführungsform des Inhalators, und
- Fig. 6: einen Schnitt durch das Unterteil einer vierten Ausführungsform des Inhalators.

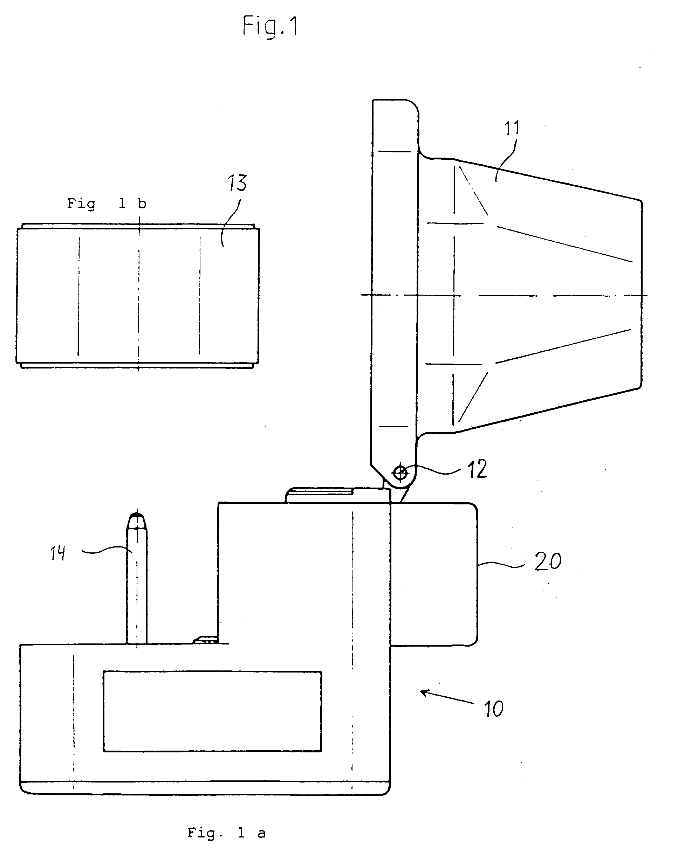

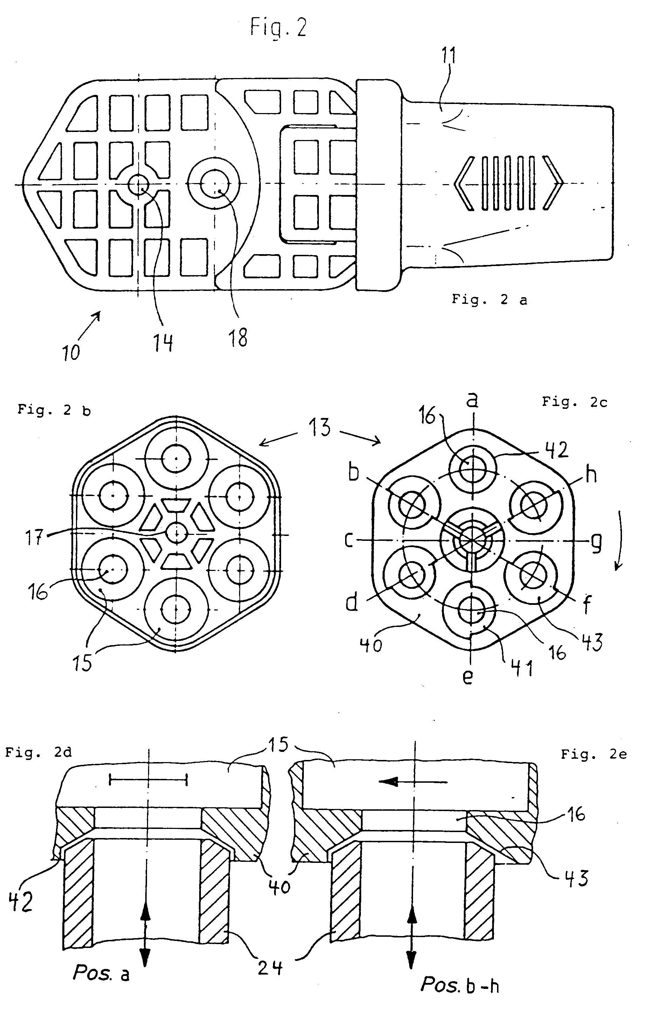

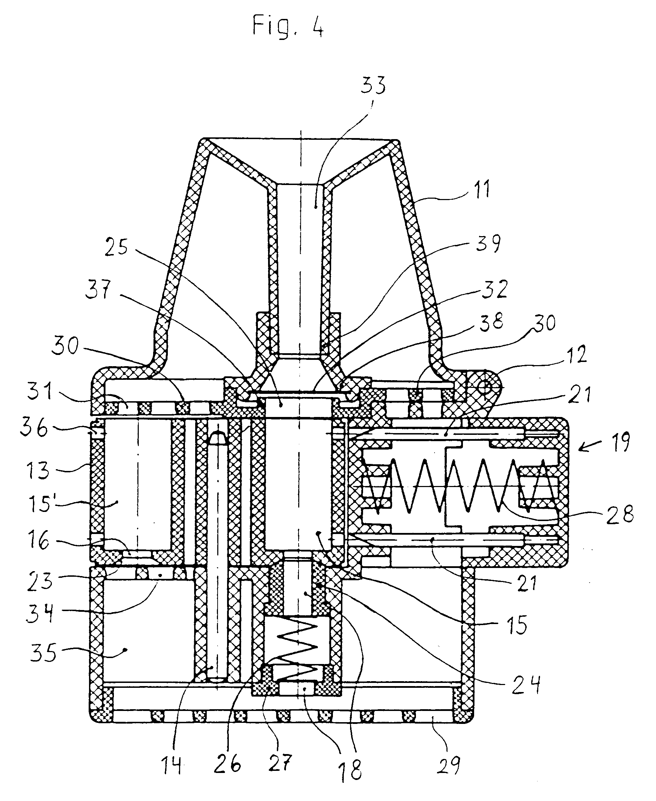

Wie aus Fig. 1a, 1b und 2a ersichtlich, besteht der Inhalator im wesentlichen aus einem Inhalatorgehäuse 10 mit einem Mundstück 11, das seitlich am oberen Rand des Inhalatorgehäuses um eine Achse 12 schwenkbar angelenkt ist. Zur Aufnahme der Kapseln dienen Kammern 15, 15' (Fig. 4) in einem Revolvermagazin 13, das auf einen exzentrisch im Inhalatorgehäuse 10 angeordneten Stift 14 aufsteckbar ist. Nach Aufstecken des Revolvermagazins 13 wird das Mundstück 11 in seine Normalstellung - als Kappe auf dem Gehäuse - gebracht; der Inhalator ist funktionsfähig. Wie aus Figur 2b ersichtlich, besitzt das Revolvermagazin 13 6 Kammern 15 zur Aufnahme der nicht dargestellten Kapseln. Der Boden jeder Kammer 15 weist eine Lufteintrittsbohrung 16 auf. Ferner besitzt das Revolvermagazin 13 eine axiale Führung 17 für den Stift 14.

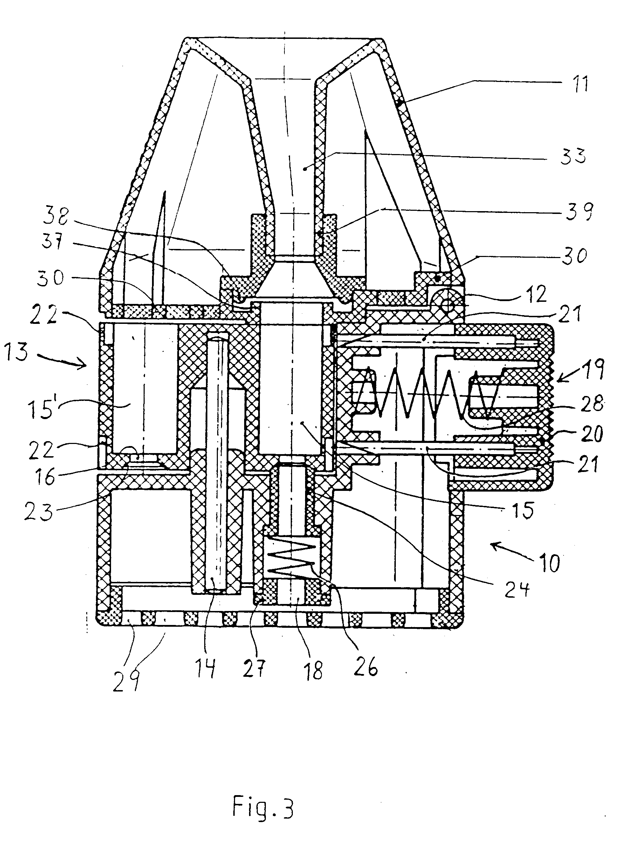

Wie aus Figur 3 ersichtlich, besitzt der Inhalator angrenzend an die unter dem Inhalationskanal 33 angeordnete Kammer 15 eine Schneideinrichtung 19, die über eine Bedienungstaste 20 zu betätigen ist. Diese Schneideinrichtung 19 weist zwei Schneiden 21 auf, die in den oberen bzw. unteren Teil der besagten Kammer 15 radial eingeführt werden können, wobei die Revolvermagazin-Außenwand zur leichteren Durchführung der Schneiden 21 an entsprechenden Stellen Durchbrüche oder geschwächte Bereiche 22 aufweist. Die Schneiden 21 dienen zum Öffnen der in einer Kammer 15 befindlichen Kapsel in der Nähe von deren oberen bzw. unteren Ende. Das Revolvermagazin 13 besitzt ferner unterhalb der Bohrungen 16 konische Ausnehmungen 23, in die ein Arretierbolzen 24 einrasten kann, sobald eine Kammer 15 koaxial mit dem Lufteinlaß 18 bzw. Inhalationskanal 33 des Inhalatorgehäuses ist.

Der Arretierbolzen ist an seinem in die Ausnehmung 23 eingreifenden Ende ebenfalls konisch gestaltet. Am gegenüberliegenden Ende ist er durch eine Feder 26 beaufschlagt, die sich auf einem im Inhalatorgehäuse lösbar befestigten Stopfen 27 abstützt. Dieser Stopfen weist ebenso wie der Arretierbolzen eine zentrale Durchgangsbohrung auf, die als Lufteinlaß 18 dient. Die lichte Weite der Kammer 15 ist etwa 1,1 bis 2 mal so groß wie der Kapseldurchmesser und die Länge der Kapselkammer (einschließlich des Luftaustritts 25) beträgt etwa das 1,1 - 1,6fache der Kapsellänge. Die lichte Weite der Kammer ist jedoch kleiner als die Länge der Kapsel.

Zur Vorbereitung des Inhalators wird bei eingelegtem Revolvermagazin 13 eine der Kammern 15 durch Drehung des Revolvermagazins in eine Position gebracht, in der die bodenseitige Bohrung 16 bzw. die konische Ausnehmung 23 koaxial zur Lufteinlaßöffnung 18 ausgerichtet ist. Die Einstellung der Kammer 15 wird durch Einrasten des Arretierbolzens 24 in die Ausnehmung 23 erleichtert. Nach dem Einrasten des Bolzens fluchten die Lufteintrittsöffnung 18 und die Bodenöffnung 16 der Kammer 15. Die Kapselkappe steht dabei auf der besagten Bodenöffnung 16 und verschließt diese. Durch Betätigung der Bedienungstaste 20 gegen die Kraft einer Feder 28 werden die Schneiden 21 radial in Richtung auf die Kammer 15 bewegt, wobei sie zunächst die geschwächten Bereiche 22 durchstoßen bzw. in passende Öffnungen in der Seitenwand des Revolvermagazins eintreten und schließlich die Kapsel oben und unten nahe ihrem Ende öffnen. Dabei dürfen die halbkugelförmigen Kappen der Kapseln nicht zerstört werden, weil sie eine Art Ventilfunktion ausüben sollen.

Zur Erleichterung des Durchstoßens der geschwächten Bereiche 22 sind die entsprechenden Stellen vorzugsweise versetzt angeordnet, so daß die obere Schneide den geschwächten Bereich durchstoßen hat, bevor die untere Schneide ihn erreicht.

Wird nunmehr Luft über das Mundstück 11 angesaugt, so versetzt die von den bodenseitigen Öffnungen 29 des Gehäuses 10 und dem Lufteinlaß 18 her in die Kammer 15 einströmende Luft die Kapsel in heftige Vibration, wirbelt das Pulver in der Kapsel auf, vermischt sich damit und wird schließlich inhaliert. Das Mundstück 11 ist im allgemeinen röhrenförmig ausgebildet, kann jedoch auch der Mundform angepaßt und abgeflacht sein. Ebenso sind in Abänderung der dargestellten Ausführungsform axiale oder in einem Winkel zur Achse der Kammer oder seitlich zur Kammerachse versetzt Mundstückanordnungen möglich.

Während bei der in Figur 3 dargestellten Ausführungsvariante das Mundstück bzw. die Kappe 11 bodenseitig mit einer im wesentlichen geschlossenen Einsatzplatte 30 versehen ist, besitzt die besagte Einsatzplatte 30 nach Figur 4 Durchbrechungen 31. Ferner ist bei der in Figur 4 dargestellten Ausführungsvariante der Anfang des Inhalationskanals 33 mit einem Sieb 32 bedeckt, welches verhindert, daß die Kapsel oder Kapselbruchstücke beim Inhalieren in den Inhalationskanal 33 im Mundstück gelangen. Alternativ hierzu können an der besagten Stelle Wandvorsprünge vorgesehen sein, welche die Kapsel zurückhalten. Die Siebplatte 32 ist vorzugsweise im Zentrum der Einsatzplatte 30 angeordnet, vorteilhaft im Klemmsitz zwischen einem den Luftdurchlaß umfassenden Anschlag 37 der Platte 30 und dem Rand einestrichterförmigen Verbindungsstückes 38, welches auf den Anfang 39 des Inhalationskanals 33 so aufgesteckt ist, daß der Trichterrand der Einsatzplatte 30 zugewandt ist und mit dieser im Eingriff steht. Dort können auch die alternativ vorgesehenen Vorsprünge angeordnet sein.

Weiterhin kann das Inhalatorgehäuse 10 in der dem Revolvermagazin 13 bzw. dessen Bohrungen 16 zugewandten Seite Durchbrechungen 34 aufweisen, die in einen ringförmigen Gehäusehohlraum 35 münden, der mit dem bodenseitigen Lufteinlaß 18 in Verbindung stehen kann.

In den Fig. 2c, d und e ist eine bevorzugte Ausführungsform der Arretierung des Revolvermagazins dargestellt. Fig. 2c zeigt eine Ansicht der Bodenplatte 40 des Revolvermagazins 13. Fig. 2d und 2e zeigen vergrößerte Teilschnitte der Eingriffsposition des Arretierbolzens 24 in die Bodenplatte 40 der bevorzugten Ausführungsform der Bodenplatte 40 nach Fig. 2c. Wie aus Fig. 2c ersichtlich, ist um jede Bohrung 16 eine mit den Bezugsziffern 43 und 41 bezeichnete konzentrische Fläche angeordnet. Diese Fläche soll die Austrittskontur der trichterförmigen Erweiterung der Bohrung 16 in der Bodenplatte 40 darstellen. Aus Fig. 2e ist deutlicher die besondere Gestaltung dieser Austrittskontur zu ersehen. Sie weist einerseits eine Schräge 43 auf, die glatt in der Oberfläche der Bodenplatte 40 ausläuft und auf der gegenüberliegenden Seite eine Kante 42 (Fig. 2d), die einen kleineren Radius beschreibt. Die Kante 42 stellt einen Anschlag für den Arretierbolzen 24 dar und verhindert ein Verdrehen des Magazins gegen die Kante 42. Das Magazin kann jedoch ohne weiteres in Richtung der Schräge 43 gedreht werden. Wie Fig. 2c zeigt, sind die über den halben Umfang der konischen Erweiterung sich erstreckenden Anschlagskanten 42 gleichsinnig angeordnet, d.h. sie sperren das Verdrehen des Magazins in der gleichen Richtung und erlauben nur die Verdrehung in Pfeilrichtung über die Schrägen 43, die in Fig. 2c mit einem größeren Durchmesser dargestellt sind. Die in Fig. 2c dargestellten Positionen sind mit a bis h bezeichnet. Die konische Ausnehmung in Position a weist die Besonderheit auf, daß sie eine über den ganzen Umfang sich erstreckende Anschlagskante 42 hat. In der Position a würde also der Arretierbolzen 24 ein Verdrehen des Magazins in keiner Richtung erlauben. Für den Benutzer stellt daher diese Position die Endposition dar, in der alle im Magazin befindlichen Kapseln verbraucht sind und ein Austausch gegen frische Kapseln erfolgen muß. In den Positionen b bis h sind die Ausnehmungen in der soeben beschriebenen Weise ausgestaltet, in der ein Drehen des Magazines nur in der Pfeilrichtung möglich ist. Auf diese Weise wird verhindert, daß eine schon einmal benutzte Kapsel wieder in den Inhalationsraum eingeschwenkt wird.

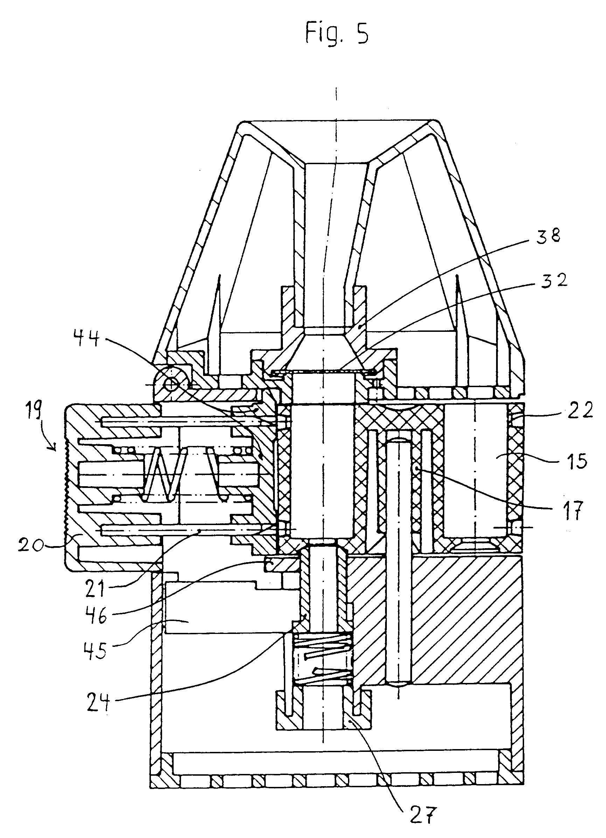

Fig. 5 zeigt eine Ausführungsform des Inhalators mit federnd gelagerter Dichtungsplatte 44 für die Schneiden 21 und einer Sperre 45 für die Bedienungstaste 20. Die Führung der Schneiden 21 in einer vom Gehäuse abgekoppelten, separaten Dichtungsplatte 44 verbessert und erleichtert das Abdichten des Raumes der Schneidvorrichtung gegenüber dem Inhalationsraum. Die Dichtungsplatte 44 kann durch die gleiche Feder, die eine Rückstellung der Betätigungstaste 20 der Schneidvorrichtung 19 bewirkt, an ihre Dichtflächen angedrückt werden.

Die Sperre 45 für die Betätigungstaste 20 ist vorzugsweise mit dem Arretierbolzen 24 verbunden. Sie entfaltet ihre Sperrwirkung, wenn der Arretierbolzen 24 bei entnommenem Revolvermagazin 13 in seine obere Anschlagsposition bei dem Anschlag 46 gelangt ist. Beim Einsetzen des Revolvermagazins wird der Arretierbolzen 24 wieder nach unten gedrückt und damit auch die Zunge 45 aus dem Sperrbereich entfernt.

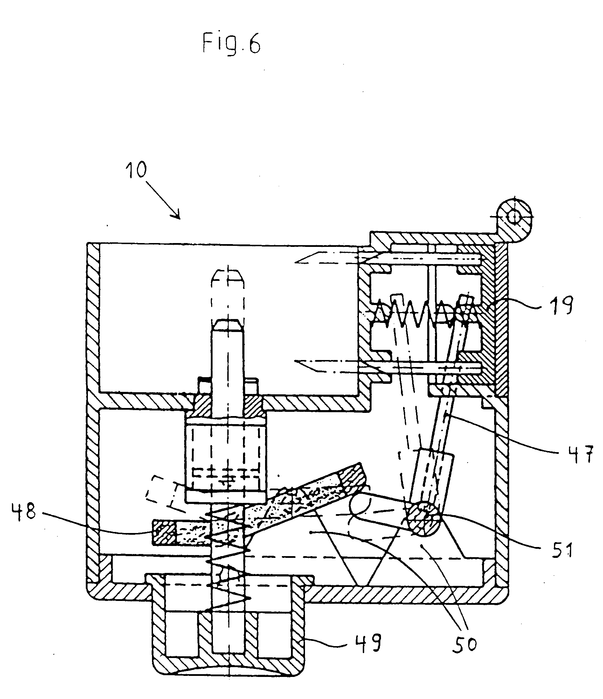

Die in Fig. 6 gezeigte Ausführungsform zeigt ein Hebelsystem 47, 48 zur Betätigung der Schneidvorrichtung 19 mit Hilfe einer im Boden des Gehäuses angeordneten Betätigungstaste 49. Der als Wippe ausgebildete erste Hebel 48 drückt beim Hochschieben der Betätigungstaste 49 auf das kurze Ende des Kniehebels 47, dessen langes Ende an der Schneidvorrichtung 19 angreift. Wippe 48 und Kniehebel 47 sind in der dargestellten Ausführungsform in Achsenhaltern 50 um die Achsen 51 schwenkbar gelagert. Diese Ausführungsform kann auch so gestaltet werden, daß durch Betätigung der Taste 49 gleichzeitig das Kapselmagazin 13 gedreht und anschließend die Schneidvorrichtung 19 in Aktion gesetzt wird.

### Bezugszeichenliste

- 10: Inhalatorgehäuse
- 11: Mundstück, Kappe
- 12: Schwenkachse
- 13: Revolvermagazin
- 14: Exzentrisch angeordneter Stift
- 15, 15': Kammern
- 16: Bohrungen
- 17: axiale Führung
- 18: Lufteinlaß
- 19: Schneideinrichtung
- 20: Bedienungstaste
- 21: Schneiden
- 22: geschwächter Bereich bzw. Durchbruch
- 23: konische Ausnehmung = Kegelmantelschräge
- 24: Arretierbolzen
- 25: Luftaustritt bzw. oberer Teil der Kammer
- 26: Feder
- 27: Stopfen
- 28: Feder für Schneideinrichtung
- 29: bodenseitige Öffnungen des Gehäuses 10
- 30: Einsatzplatte
- 31: Durchbrechungen
- 32: Sieb
- 33: Inhalationskanal
- 34: Durchbrechungen
- 35: Gehäusehohlraum
- 36: Durchbrechungen im Revolvermagazin
- 37: Anschlag
- 38: trichterförmiges Verbindungsstück
- 39: Anfang des Inhalationskanals
- 40: Bodenplatte des Revolvermagazins
- 41: Anschlagseite der konischen Ausdehnung 23
- 42: Anschlagkante der konischen Ausdehnung 23
- 43: Gleitschräge der konischen Ausdehnung 23
- 44: Dichtungsplatte
- 45: Lochungssperre
- 46: Anschlag für Lochungssperre
- 47: Kniehebel
- 48: Wippe
- 49: Hebelbetätigungstaste
- 50: Achsenhalter
- 51: Achse

## Patentansprüche

1. Inhalator für die Inhalation pulverförmiger, insbesondere mikronisierter Arzneimittel aus Kapseln, in dessen Gehäuse für die Aufnahme der Kapseln eine rohrförmige Kammer mit einem bodenseitigen Lufteinlaß und einem in ein Inhalationsmundstück mündenden Luftauslaß am gegenüberliegenden Kammerende und eine Schneideinrichtung mit zwei in den Kammerinnenraum bewegbaren Schneiden zum Öffnen der Kapseln in der Nähe von deren oberen und unteren Ende vorgesehen sind,
**gekennzeichnet durch**
ein in dem Inhalatorgehäuse (10) drehbar angeordnetes Revolvermagazin (13) mit mehreren mit jeweils einer Kapsel bestückbaren rohrförmigen Kammern (15, 15'), deren Längsachsen parallel zur Achse (14) des Revolvermagazins (13) und parallel zur Längsachse des Inhalators (10) stehen, und die zusammen mit dem Revolvermagazin (13) so angeordnet sind, daß sie zwischen den im wesentlichen zentral im Gehäuse (10) angeordneten Lufteinlaß (18) und Luftaustritt (25) geschwenkt werden können und damit eine koaxiale Einheit bilden.

2. Inhalator nach Anspruch 1,
**dadurch gekennzeichnet**,
daß das Mundstück (11) mit dem Gehäuse (10) lösbar bzw. schwenkbar verbunden ist und bei gelöstem Mundstück das Revolvermagazin (13) auf eine Stiftachse (14) aufsteckbar bzw. davon abziehbar ist.

3. Inhalator nach Anspruch 1 oder 2,
**dadurch gekennzeichnet**,
daß das Revolvermagazin (13) den Kapselkammern (15) jeweils zugeordnete Ausnehmungen (23) für einen in dem Inhalatorgehäuse (10) angeordneten, federnd gelagerten Arretierbolzen (24) aufweist, wobei die Ausnehmungen (23) so angeordnet sind, daß der vorzugsweise an seinem freien Ende konisch ausgebildete Arretierbolzen (24) nur dann dort einrastet, wenn eine der Kammern (15) eine koaxiale Einheit mit dem Luftein- und Auslaß (18) bzw. (25) bildet.

4. Inhalator nach Anspruch 3,
**dadurch gekennzeichnet,**
daß die Ausnehmungen (23) bodenseitig in der Bodenplatte (40) des Magazins (13) konzentrisch zu Lufteintrittsbohrungen (16) der Kapselkammern (15) angeordnet und wie der Mantel eines mit der Basis nach außen gewandten flachen Kegelstumpfes gestaltet sind.

5. Inhalator nach Anspruch 4,
**dadurch gekennzeichnet,**
daß eine der Ausnehmungen (23) an der Basis des Kegelstumpfmantels, aber noch in der Bodenplatte (40), eine umlaufende Anschlagskante (42) aufweist und alle übrigen Ausnehmungen (23) nur auf höchstens dem halben Umfang der Basis des Kegelstumpfmantels besagte Anschlagskante (42) aufweisen, die gleichsinnig wirkend angeordnet sind.

6. Inhalator nach einem der Ansprüche 3 bis 5,
**dadurch gekennzeichnet,**
daß der Arretierbolzen (24) eine Durchgangsbohrung aufweist, die den bodenseitigen Lufteinlaß (18, 16) bildet, wobei der Arretierbolzen (24) durch eine Feder (26) beaufschlagt ist, deren anderes Ende auf einem im Inhalatorgehäuse (1 ) lösbar befestigten Stopfen (27) aufliegt, der ebenfalls eine zentrale Durchgangsbohrung aufweist.

7. Inhalator nach einem der Ansprüche 1 bis 6,
**dadurch gekennzeichnet**,
daß die Kammerwände zur Durchführung der Schneiden (21) der Schneideinrichtung (19) radiale Durchbrechungen (36) oder zumindest geschwächte Bereiche (22) aufweisen, wobei die Schneiden (21) so angeordnet sind, daß die eine Schneide in der Nähe des Bodens der Kammer (15) und in der Nähe des Lufteinlaßes und die zweite Schneide in der Nähe des oberen Kapselendes und in der Nähe des Luftauslaßes in die Kammer (15) eintritt.

8. Inhalator nach einem der vorhergehenden Ansprüche,
**dadurch gekennzeichnet,**
daß die Schneiden (21) der Schneidvorrichtung (19) in einer federnd gelagerten Dichtungsplatte (44) geführt sind.

9. Inhalator nach einem der vorhergehenden Ansprüche,
**dadurch gekennzeichnet,**
daß am Arretierbolzen (24) eine Zunge (45) befestigt ist, die sich bis zu einem Anschlag an der Innenseite der Bedienungstaste (20) erstreckt, wenn der Arretierbolzen (24) bei entnommenem Revolvermagazin (13) seine obere Anschlagsposition (46) einnimmt, so daß die Zunge, (45) in dieser Position als Sperre für die Schneideinrichtung (19) wirkt.

10. Inhalator nach einem der Ansprüche 1 bis 9,
**dadurch gekennzeichnet**,
daß das Mundstück (11) um eine senkrecht zur Inhalatorlängsachse liegende Achse (12) schwenkbar am Inhalatorgehäuserand angelenkt ist.

11. Inhalator nach einem der Ansprüche 1 bis 10,
**dadurch gekennzeichnet,**
daß im oberen Bereich der Kammer (15), dort wo sie in den Inhalationskanal übergeht, eine Siebplatte (32) angeordnet ist, die Teil eines trichterförmigen Verbindungsstückes (38) ist, welches auf den Anfang (39) des Inhalationskanales (33) so aufsteckbar ist, daß der Trichterrand mit der Siebplatte (32) in eine Einsatzplatte (30) eingreift, die den Boden des Mundstückes (11) bildet, oder daß die Siebplatte (32) im Klemmsitz zwischen dem Trichterrand des Verbindungsstückes (38) und einem Anschlag (37) der Einsatzplatte (30) austauschbar befestigt ist.

12. Inhalator nach einem der Ansprüche 1 bis 11,
**dadurch gekennzeichnet**,
daß die lichte Weite einer Kammer (15, 15') etwa 1,1 - 2 mal so groß wie der Kapseldurchmesser und die gesamte Länge der Kapselkammer (15, 25) etwa das 1,1 - 1,6fache der Kapsellänge beträgt.

13. Inhalator nach einem der vorhergehenden Ansprüche,
**dadurch gekennzeichnet,**
daß die Schneideinrichtung (19) mit Hilfe eines Hebelsystems (47, 48) und einer im Boden des Gehäuses (10) angebrachten Betätigungstaste (49) betätigbar ist.

14. Inhalator nach Anspruch 13,
**dadurch gekennzeichnet,**
daß die Betätigungstaste (49) in Wirkverbindung mit dem einen Ende einer Wippe (48) steht, deren anderes Ende auf das eine Ende eines Kniehebels (47) einwirkt, dessen anderes Ende mit der Schneidvorrichtung (19) verbunden ist.

## Claims

1. Inhaler for the inhalation of powdered, particularly micronised drug preparations from capsules, the housing of said inhaler containing a tubular chamber for receiving the capsules, having an air inlet at the bottom, and an air outlet at the opposite end of the chamber merging into an inhalation mouthpiece, and a cutting device having two blades which are movable into the interior of the chamber for opening the capsules in the region of the top and bottom ends thereof, characterised by a revolver magazine (13) rotatably mounted in the inhaler housing (10), having a plurality of tubular chambers (15, 15'), each of which can be loaded with a capsule, the longitudinal axis of said chambers being parallel to the axis (14) of the revolver magazine (13) and parallel to the longitudinal axis of the inhaler (10), and which are arranged together with the revolver magazine (13) in such a way that they can be pivoted into position between the air inlet (18) and the air outlet (25) arranged substantially centrally in the housing (10) and thus form a coaxial unit.

2. Inhaler according to claim 1, characterised in that the mouthpiece (11) is releasably or pivotally connected to the housing (10) and, with the mouthpiece detached, the revolver magazine (13) can be fitted on to or removed from a pin (14).

3. Inhaler according to claim 1 or 2, characterised in that the revolver magazine (13) has recesses (23) associated with each of the capsule chambers (15), for a locking bolt (24) resiliently mounted in the inhaler housing (10), the recesses (23) being arranged so that the locking bolt (24), which is preferably of conical construction at its free end, only engages therein if one of the chambers (15) forms a coaxial unit with the air inlet and outlet (18 and 25), respectively.

4. Inhaler according to claim 3, characterised in that the recesses (23) are provided at the bottom in the base plate (40) of the magazine (13), concentrically with air inlet bores (16) in the capsule chambers (15), and are shaped in the manner of the exterior of a flat truncated cone with its base facing outwards.

5. Inhaler according to claim 4, characterised in that one of the recesses (23) has an encircling abutment edge (42) on the base of the truncated cone shell, but still in the base plate (40), and all the other recesses (23) have the said abutment edge (42) over at most half the circumference of the base of the truncated cone shell, said abutment edges (42) acting in the same direction.

6. Inhaler according to one of claims 3 to 5, characterised in that the locking bolt (24) has a through-bore which forms the air inlet (18, 16) at the bottom, the locking bolt (24) being acted upon by a spring (26), the other end of which abuts on a plug (27) releasably secured in the inhaler housing (10), this plug (27) also having a central through bore.

7. Inhaler according to one of claims 1 to 6, characterised in that the walls of the chamber have radial openings (36) or at least frangible regions (22) for the passage of the blades (21) of the cutting device (19), the blades (21) being arranged so that one blade enters near the bottom of the chamber (15) near the air inlet and the second blade enters the chamber (15) near the top end of the capsule and near the air outlet.

8. Inhaler according to one of the preceding claims, characterised in that the blades (21) of the cutting device (19) are guided in a resiliently mounted sealing plate (44).

9. Inhaler according to one of the preceding claims, characterised in that, secured to the locking bolt (24), there is a tongue (45) which extends as far as a stop on the inside of the operating button (20), when the locking bolt (24) assumes its upper position of abutment (46), with the revolver magazine (13) removed, so that the tongue (45) in this position acts as a barrier for the cutting device (19).

10. Inhaler according to one of claims 1 to 9, characterised in that the mouthpiece (11) is hinged to the edge of the inhaler housing so as to be pivotable about an axis (12) extending perpendicularly to the longitudinal axis of the inhaler.

11. Inhaler according to one of claims 1 to 10, characterised in that, in the upper region of the chamber (15), where it merges with the inhalation channel, a sieve plate (32) is provided which is part of a funnel shaped connecting member (38) which can be fitted on to the start (39) of the inhalation channel (33) in such a way that the funnel edge with the sieve plate (32) engages in an insert plate (30) which forms the base of the mouthpiece (11), or the sieve plate (32) is replaceably clamped between the funnel edge of the connecting member (38) and a stop (37) of the insert plate (30).

12. Inhaler according to one of claims 1 to 11, characterised in that the internal width of a chamber (15, 15') is about 1.1. to 2 times as great as the capsule diameter and the overall length of the capsule chamber (15, 25) is about 1.1 to 1.6 times the capsule length.

13. Inhaler according to one of the preceding claims, characterised in that the cutting device (19) can be actuated by means of a lever system (47, 48) and an operating button (49) provided in the base of the housing (10).

14. Inhaler according to claim 13, characterised in that the operating button (49) is operatively connected to one end of a rocker arm (48), the other end of which acts on one end of a toggle lever (47), the other end of which is connected to the cutting device (19).

## Revendications

1. Inhalateur pour l'inhalation de médicaments pulvérulents, en particulier micronisés, à partir de capsules, dans l'enveloppe duquel sont prévus une chambre tubulaire pour la réception des capsules, avec une entrée d'air au fond et une sortie d'air débouchant dans une embouchure d'inhalation à l'extrémité opposée de la chambre, et un dispositif de coupure comportant deux lames mobiles dans l'espace intérieur de la chambre pour l'ouverture des capsules au voisinage de leurs extrémités supérieure et inférieure, caractérisé par un magasin revolver (13) disposé de façon à pouvoir tourner dans l'enveloppe (10) de l'inhalateur et comportant plusieurs chambres tubulaires (15), (15') pouvant recevoir chacune une capsule, dont l'axe longitudinal est parallèle à l'axe (14) du magasin revolver (13) et parallèle à l'axe longitudinal de l'inhalateur (10), et qui sont disposées avec le magasin revolver (13) de façon à pouvoir être amenées par rotation entre l'entrée d'air (18) et la sortie d'air (25) disposées essentiellement au centre de l'enveloppe (10), et à former ainsi une unité coaxiale.

2. Inhalateur selon la revendication 1, caractérisé en ce que l'embouchure (11) est reliée à l'enveloppe (10) de façon mobile ou pivotante et en ce que, lorsque l'embouchure est détachée, le magasin revolver (13) peut être monté sur un ce de broche (14) ou en être retiré.

3. Inhalateur selon la revendication 1 ou 2, caractérisé en ce que le magasin revolver (13) comporte des évidements (23) correspondant à chacune des chambres de capsules (15) pour une cheville de blocage (24) montée sur ressort dans l'enveloppe (10) de l'inhalateur, les évidements (23) étant disposés de façon que la cheville de blocage (24), de préférence de forme conique à son extrémité libre, ne s'y enclenche que lorsque l'une des chambres (15) forme avec l'entrée et la sortie d'air (18) et (25) une unité coaxiale.

4. Inhalateur selon la revendication 3, caractérisé en ce que les évidements (23) sont disposés au fond dans la plaque de fond (40) du magasin (13) de façon concentrique par rapport aux orifices d'entrée d'air (16) des chambres de capsules (15) et ont la forme d'un cône tronqué aplati dont la base est tournée vers l'extérieur.

5. Inhalateur selon la revendication 4, caractérisé en ce que l'un des évidements (23) présente à la base du cône tronqué, mais encore dans la plaque de fond (40), une arête de butée tournante (42), et tous les autres évidements (23) ne présentent ladite arête de butée (42) qu'au plus sur la moitié du périmètre de la base du cône tronqué, et disposée de façon à agir dans le même sens.

6. Inhalateur selon l'une des revendications 3 à 5, caractérisé en ce que la cheville de blocage (24) présente un trou de passage qui forme l'entrée d'air du fond (18, 16), la cheville de blocage (24) étant percutée par un ressort (26) dont l'autre extrémité s'appuie sur un bouchon (27) fixé de façon amovible dans l'enveloppe de l'inhalateur (10) et qui comporte aussi un trou de passage central.

7. Inhalateur selon l'une des revendications 1 à 6, caractérisé en ce que les parois des chambres comportent des découpures radiales (36) ou au moins des zones amincies (22) pour le passage des lames (21) du dispositif de coupure (19), les lames (21) étant placées de façon qu'une des lames pénètre à proximité du fond de la chambre (15) et à proximité de l'entrée d'air, et que l'autre lame pénètre à proximité de l'extrémité supérieure de la capsule et à proximité de la sortie d'air dans la chambre (15).

8. Inhalateur selon l'une des revendications précédentes, caractérisé en ce que les lames (21) du dispositif de coupure (19) sont guidées dans une plaque d'étanchéité (44) montée sur ressort.

9. Inhalateur selon l'une des revendications précédentes, caractérisé en ce que, sur la cheville de blocage (24), est fixée une languette (45) qui avance jusqu'à une butée au niveau de la face interne de la touche de commande (20) lorsque la cheville de blocage (24) occupe sa position d'arrêt supérieure (46) quand le magasin revolver (13) est retiré, de sorte que la languette (45) dans cette position a un effet de blocage pour le dispositif de coupure (19).

10. Inhalateur selon l'une des revendications 1 à 9, caractérisé en ce que l'embouchure (11) est articulée au bord de l'enveloppe de l'inhalateur de façon à pouvoir pivoter autour d'un axe (12) perpendiculaire à l'axe longitudinal de l'inhalateur.

11. Inhalateur selon l'une des revendications 1 à 10, caractérisé en ce que, dans la zone supérieure de la chambre (15), là où elle passe dans le canal d'inhalation, est disposée une plaque de tamisage (32) qui fait partie d'une pièce de jonction (38) en forme d'entonnoir qui s'emboîte sur le début (39) du canal d'inhalation (33) de façon que le bord de l'entonnoir s'engage avec la plaque de tamisage (32) dans une plaque intercalaire (30) qui forme le fond de l'embouchure (11), ou de façon que la plaque de tamisage (32) se fixe de manière interchangeable dans le logement de blocage entre le bord de l'entonnoir de la pièce de jonction (38) et une butée (37) de la plaque intercalaire (30).

12. Inhalateur selon l'une des revendications 1 à 11, caractérisé en ce que le diamètre intérieur d'une chambre (15, 15') est environ 1,1 à 2 fois plus grand que le diamètre d'une capsule et en ce que la longueur totale des la chambre de capsule (15, 25) représente environ 1,1 à 1,6 fois la longueur de la capsule.

13. Inhalateur selon l'une des revendications précédentes, caractérisé en ce que le dispositif de coupure (19) peut être commandé à l'aide d'un système de levier (47, 48) et d'une touche de commande (49) montée dans le fond de l'enveloppe (10).

14. Inhalateur selon la revendication 13, caractérisé en ce que la touche de commande (49) est raccordée de façon active à l'une des extrémités d'une bascule (48) dont l'autre extrémité agit sur l'une des extrémités d'un levier coudé (47) dont l'autre extrémité est reliée au dispositif de coupure (19).
